# EUROPÄISCHE PATENTANMELDUNG

(11) **EP 1 103 235 A2**
(43) Veröffentlichungstag der Anmeldung: **30.05.2001**
(21) Anmeldenummer: 00126075.1
(22) Anmeldetag: 29.11.2000
(51) Int. Cl.: A61F 2/44

(54) **Cageelement, sowie Satz von Cageelementen**

(30) Priorität: 29.11.1999 DE 19957339
(71) Anmelder: Copf, Franz, D-70178 Stuttgart (DE)
(72) Erfinder: Copf, Franz, D-70178 Stuttgart (DE)
(74) Vertreter: Ostertag, Reinhard

(57) **Zusammenfassung**

Um die Knochenbildung zwischen den Wirbelkörpern durch ein Cageelement hindurch zu optimieren, wird erfindungsgemäß ein Cageelement (10) mit einem Kopfabschnitt (12), einem Zentralabschnitt (14) und einem Bodenabschnitt (16) vorgeschlagen, dessen Zentralabschnitt (14) eine Mehrzahl von sich vom Kopfabschnitt (12) bis zum Bodenabschnitt (16) erstreckenden Streben (26, 28, 30) umfaßt. Die Streben (26, 28, 30) sind so schmal, daß die durch sie abgedeckte Umfangserstreckung maximal 25% der gesamten Umfangserstreckung des Zentralabschnitts (14) entspricht.

## Beschreibung

Die vorliegende Erfindung betrifft ein Cageelement nach dem Oberbegriff des Anspruchs 1, sowie einen Satz von Cageelementen.

Unter einem Cageelement versteht man einen insgesamt in etwa zylindrischen Körper, welcher im Intradiskalraum, also dem Bandscheibenfach, zwischen zwei Wirbelkörpern anstelle der menschlichen Bandscheibe positioniert wird, um den Abstand zwischen den beiden Wirbelkörpern sicherzustellen.

Ein vom Markt her bekanntes Cageelement hat die Form einer zylindrischen Hülse, in deren Umfangswand an mehreren Stellen Fenster ausgeschnitten sind. An ihren Stirnseiten ist die Hülse offen, so daß der Operateur Spongiosa in das Innere der Hülse einbringen kann. Hierdurch soll das Knochenwachstum zwischen den beiden Wirbelkörpern gefördert werden. Im Idealfall sind nach einiger Zeit die beiden Wirbelkörper einstückig durch Knochen miteinander verbunden.

Bei dem bekannten Cageelement wurde festgestellt, daß das Knochenwachstum von einem Wirbelkörper zum anderen durch den Innenraum des Cageelements hindurch oft nicht zufriedenstellend verläuft. Darüber hinaus kam es vor, daß sich das bekannte Cageelement aus seiner vom Operateur bestimmten Position im Laufe der Zeit gelöst hat und hierdurch das Operationsergebnis gefährdet wurde.

Die vorliegende Erfindung hat daher die Aufgabe, ein Cageelement zu schaffen, welches zum einen den notwendigen Abstand zwischen zwei Wirbelkörpern sicherstellt, welches auf der anderen Seite jedoch die Knochenbildung zwischen benachbarten Wirbelkörpern möglichst wenig behindert oder sogar fördert und im Intradiskalraum verdrehsicher gehalten ist.

Diese Aufgabe wird durch ein Cageelement mit den im Anspruch 1 angegebenen Merkmalen gelöst.

Bei dem erfindungsgemäßen Cageelement wird auf die bekannte hülsenartige Form bewußt verzichtet. Es wurde nämlich festgestellt, daß die meisten Kräfte zwischen zwei Wirbelkörpern einerseits an der ventralen Zirkumferenz und andererseits an der dorsalen Zirkumferenz der Wirbelkörper übertragen werden. Der Zentralabschnitt des Cageelements muß also nur vergleichsweise geringe Kräfte übertragen, wenn der Kopfabschnitt und der Bodenabschnitt dafür ausreichend stabil sind und an den jeweiligen Zirkumferenzen anliegen. Somit kann der Zentralabschnitt im Vergleich zum Kopfabschnitt und zum Bodenabschnitt weniger stabil ausgebildet sein und einen im Vergleich zu dem bekannten Cageelement großflächigeren direkten Weg von einem Wirbelkörper zum anderen freigeben. Dies wird durch die erfindungsgemäßen Streben erreicht. Auf Grund des direkten Wegs zwischen den Wirbelkörpern ist in diesem Bereich ein besserer Kraftschluß z.B. durch eingebrachte Spongiosa durch das Cageelement hindurch möglich, wodurch die Knochenbildung beschleunigt wird. Durch die schmalen Streben ist ferner eine bessere Röntgenkontrolle während und nach dem Eingriff möglich, da die Abschattung durch für Röntgenstrahlen undurchlässige Teile geringer ist.

Bei einem solchen erfindungsgemäßen Cageelement werden zwar die Hauptkräfte über den Kopfabschnitt und den Bodenabschnitt übertragen, die an der Kompakta der Wirbelkante anliegen; es kann jedoch durch die in den Mittelabschnitt eingebrachte Spongiosa zu einem erheblichen direkten Kraftschluß zwischen den beiden Wirbelkörpern kommen, was die Knochenbildung positiv beeinflußt.

Aufgrund der Streben ist außerdem die Umfangsfläche der Zentralabschnitte nicht "glatt", was wie eine Verdrehsperre wirkt und somit das Cageelement in seiner Position sichert.

Vorteilhafte Weiterbildung der Erfindung sind in den Unteransprüchen angegeben.

Gemäß der Weiterbildung nach Anspruch 2 umfaßt der Bodenabschnitt sich im wesentlichen radial erstreckende Verbindungselemente, welche an ihrem radial äußeren Ende mit den Enden der Streben und mit ihrem radial inneren Ende miteinander verbunden sind. Diese Weiterbildung bietet zwei wesentliche Vorteile: Durch die "unrunde" Ausgestaltung des Bodenabschnittes ist das Cageelement im Einbauzustand noch besser gegen ein unbeabsichtigtes Sichverdrehen gesichert; es ist also sicher in seiner vom Operateur gewünschten Position zwischen den beiden Wirbelkörpern verankert. Hierdurch wird die Gefahr einer späteren Verschlechterung des Operationsergebnisses durch ein Verschieben oder Herausdrehen des Cageelements aus der vom Operateur gewünschten Position sicher verhindert.

Zum anderen bietet der solchermaßen gestaltete Bodenabschnitt nur eine relativ geringe Abstützfläche gegenüber den Wirbelkörpern, wodurch wegen der hohen Flächenpressung während eines gewissen Zeitraums nach der Operation die Streben und die Stirnseiten der Verbindungselemente etwas in die Knochenwand der Wirbelkörper einsinken. Dies bewirkt eine optimale Verankerung des erfindungsgemäßen Cageelements zwischen den beiden Wirbelkörpern und erleichtert den Kraftschluß durch das Cageelement hindurch, wodurch die Knochenbildung weiterhin positiv beeinflußt wird.

Gemäß Anspruch 3 schneiden die Längsachsen der Verbindungselemente die Längsachse des Cageelements. Sind an dem Cageelement z.B. nur zwei Streben vorgesehen, würde ein solches Verbindungselement einfach diagonal von einem Strebenende zum anderen verlaufen. Bei drei Streben würden die Verbindungselemente einen Stern bilden, dessen Zentralachse mit der Längsachse des Cageelements fluchtet. Hierbei handelt es sich um besonders stabile Weiterbildungen des erfindungsgemäßen Cageelements, welche die zwischen zwei Wirbelkörpern auftretenden Kräfte besonders gut aufnehmen können.

Das in Anspruch 4 angegebenen Cageelement weist eine ungleiche Anzahl von Verbindungselementen auf, deren radial äußere Stirnflächen in Anlage an gegenüberliegende Wirbelkörper kommen können, wobei die Gesamtfläche der an dem einen Wirbelkörper anlegbaren radial äußeren Stirnflächen in etwa gleich der Gesamtfläche der an dem anderen Wirbelkörper anlegbaren radial äußeren Stirnflächen ist.

Besonders vorteilhaft bei einem Cageelement, dessen Zentralabschnitt drei Streben aufweist, ist es gemäß Anspruch 5, wenn die radial äußere Stirnfläche eines Verbindungselements in etwa gleich groß ist wie die Summe der radial äußeren Stirnflächen der beiden anderen Verbindungselemente.

Mit in den Ansprüchen 4 und 5 angegebenen Weiterbildungen wird sichergestellt, daß der Bodenabschnitt des erfindungsgemäßen Cageelements den beiden vom Cageelement auseinanderzuhaltenden Wirbelkörpern in etwa dieselbe Auflagefläche bietet.

Eine weitere Möglichkeit, den Bodenabschnitt zu stabilisieren und ggf. die Anlageflächen zu vergrößern, ist in Anspruch 6 angegeben, wonach der Bodenabschnitt Verbindungselemente aufweist, welche die Enden benachbarter Streben miteinander im wesentlichen geradlinig verbinden.

Selbstverständlich kann der Bodenabschnitt jedoch auch einen Bodenring umfassen, der mit den Enden der Streben verbunden ist. Bei dieser in Anspruch 7 angegebenen Weiterbildung ist allerdings das Verdrehrisiko etwas erhöht.

Optimal ist es, wenn die Lage zweier benachbarter Wirbelkörper vom Operateur so eingestellt werden kann, daß diese nicht absolut parallel zueinander angeordnet sind, sondern der Abstand zwischen den beiden Wirbelkörpern dorsal etwas geringer ist als ventral. Eine solche Positionierung der beiden Wirbelkörper wird durch ein Cageelement gemäß Anspruch 8 ermöglicht, bei dem die kleinste lichte Außenweite des Bodenabschnitts kleiner ist als diejenige des Kopfabschnitts.

Das Einführen des Cageelements in den Intradiskalraum wird, wie in Anspruch 9 angegeben ist, dadurch erleichtert, daß auf einem zylindrischen Bereich des Kopfabschnitts ein Außengewinde vorhanden ist. Dieses kann gegebenenfalls mit einem bereits in den Intradiskalraum eingebrachten Innengewinde zusammenarbeiten.

Das Einführen des erfindungsgemäßen Cageelements wird gemäß Anspruch 10 weiter erleichtert, wenn der Kopfabschnitt an seinem den Streben entgegengesetzten Enden einen nach außen konisch zulaufenden Bereich aufweist. Hierdurch wird auch verhindert, daß beim Einführen des Cageelements Gewebe am äußeren Rand des Kopfabschnitts des Cageelements hängenbleibt und es hierdurch zu zusätzlichen Verletzungen kommt.

Vorteilhaft ist auch, wenn, wie in Anspruch 11 angegeben ist, auf der radial außen liegenden Oberfläche der Streben ein Außengewinde vorhanden ist, derart, daß das Cageelement insgesamt in eine Öffnung einschraubbar ist.

Die Erfindung betrifft auch einen Satz von Cageelementen nach einem der vorhergehenden Ansprüche, gemäß dem nebengeordneten Anspruch 12. In diesem Satz sind Cageelemente unterschiedlicher Länge, unterschiedlichen Durchmessers und mit unterschiedlichen Verhältnissen der lichten Außenweiten der Bodenabschnitte im Verhältnis zu denen der Kopfabschnitte vorhanden. Aus diesem Satz wird nach einer Vermessung der individuellen geometrischen Verhältnisse eines Patienten das passende Cageelement ausgewählt.

Nachfolgend wird die Erfindung unter Bezugnahme auf die beiliegende Zeichnung im Detail beschrieben. In dieser zeigen:
- Figur 1:: eine perspektivische Darstellung eines ersten Ausführungsbeispiels eines Cageelements;
- Figur 2:: eine Seitenansicht des Cageelements von Figur 1;
- Figur 3:: eine Draufsicht auf das Cageelement von Figur 1;
- Figur 4:: eine Schnittansicht längs der Linie IV-IV von Figur 2;
- Figur 5:: eine Seitenansicht eines zweiten Ausführungsbeispiels eines Cageelements;
- Figur 6:: eine Schnittansicht längs der Linie VI-VI von Figur 5;
- Figur 7:: eine Seitenansicht eines dritten Ausführungsbeispiels eines Cageelements; und
- Figur 8:: eine Schnittansicht längs der Linie VIII-VIII von Figur 7.

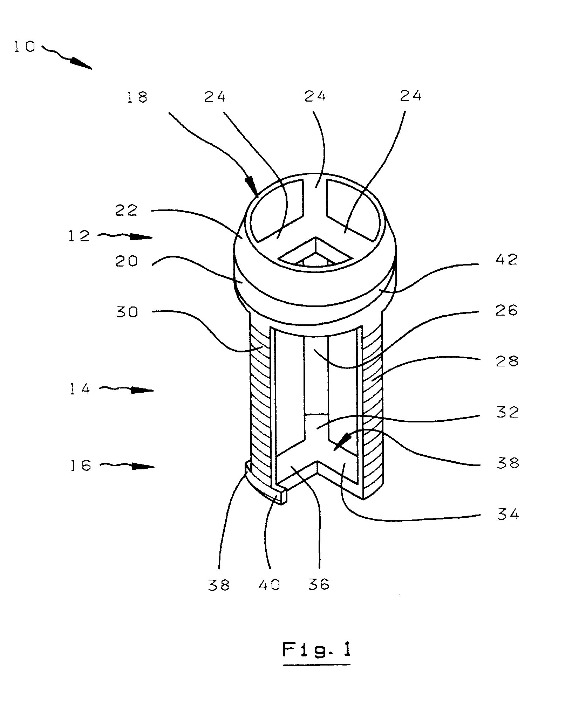

In Figur 1 trägt ein Cageelement insgesamt das Bezugszeichen 10. Es umfaßt einen ringförmigen Kopfabschnitt 12, einen an diesen angeformten Zentralabschnitt 14 und einen wiederum an diesen angeformten Bodenabschnitt 16.

Der Kopfabschnitt 12 ist folgendermaßen aufgebaut: Er umfaßt einen Einführring 18, welcher zum Zentralabschnitt 14 hin einen zylindrischen Abschnitt 20 und zum in Figur 1 oberen Ende des Cageelements 10 hin einen konischen Abschnitt 22 aufweist. Der Innendurchmesser des Einführrings 18 ist jedoch über seine Höhe konstant. Im Bereich des konischen Abschnitts 22 des Einführrings 18 sind drei Verbindungselemente 24 sternförmig im Sinne von Speichen einstückig mit der inneren Mantelfläche des Einführrings 18 zu dessen Versteifung verbunden.

Der Zentralabschnitt 14 umfaßt drei sich axial vom in Figur 1 unteren Rand des zylindrischen Abschnitts 20 axial nach unten erstreckende Streben 26, 28 und 30, welche zumindest auf ihrer Außenseite in Umfangsrichtung wie der Zylinderabschnitt 20 gekrümmt sind. Die radiale Dicke der Streben 26, 28 und 30 entspricht der Wandstärke des Zylinderabschnitts 20 des Einführrings 18.

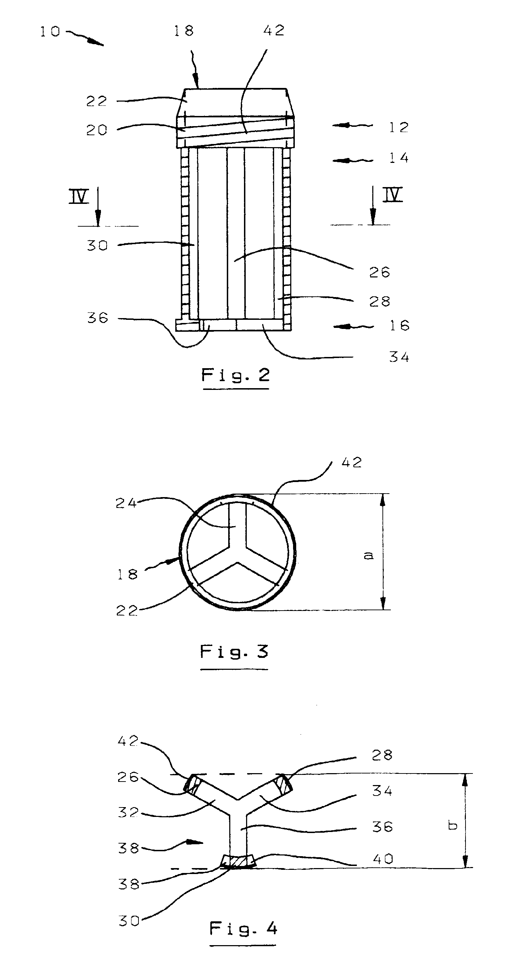

Die Streben 26, 28 und 30 sind gleichmäßig über den Umfang des Cageelements verteilt und sind so schmal, daß die durch sie abgedeckte Umfangserstreckung nur wenig mehr als 12 % der gesamten Umfangserstreckung des Zentralabschnitts entspricht. Wie besonders gut aus Figur 2 ersichtlich ist, wird hierdurch ein sehr großer freier Durchgang quer zur Längsachse des Cageelements 10 geschaffen.

Der Bodenabschnitt 16 (vergleiche insbesondere Figuren 3 und 4) umfaßt drei sich radial erstreckende Verbindungselemente 32, 34 und 36, die an ihrem radial äußeren Ende mit den Enden der Streben 26, 28 und 30 verbunden sind. Innen sind die Verbindungselemente 32, 34 und 36 miteinander im Bereich der Längsachse des Cageelements 10 verbunden, so daß sie einen Verbindungsstern 38 bilden.

Die radial äußere Stirnfläche des Verbindungselements 36 weist in Umfangsrichtung zu beiden Seiten des Verbindungselements 36 verlaufende Erweiterungen 38 und 40 auf, wodurch die Stirnfläche des Verbindungselements 36 insgesamt etwa gleich groß ist wie die Summe der radial äußeren Stirnflächen der Verbindungselemente 32 und 34. Sämtliche Stirnflächen der Verbindungselemente 32, 34 und 36, einschließlich der Erweiterungen 38 und 40 sind in Umfangsrichtung entsprechend dem Radius des Zylinderabschnitts 20 des Einführrings 18 gekrümmt.

Die lichte Außenweite b (vergl. Figur 3) des Bodenabschnitts 16, welche gleich dem Abstand zweier paralleler Ebenen ist, zwischen die der Bodenabschnitt 16 einschiebbar ist, ist deutlich kleiner als die lichte Außenweite a (vergl. Figur 3) des Kopfabschnitts 12, welche gleich dessen Durchmesser ist.

Vom zylindrischen Abschnitt 20 des Einführrings 18 über die Streben 26, 28 und 30 bis zu den Stirnflächen der Verbindungselemente 32, 34 und 36 erstreckt sich ein Gewinde 42, welches in den Figuren 1 und 2 nur schematisch durch eine schraubenförmig gewundene Linie dargestellt ist.

Das Cageelement 10 wird folgendermaßen verwendet:

Vorzugsweise wird von dorsal der Intradiskalraum mit einem entsprechenden Werkzeug dilatiert, die Bandscheibe ausgeräumt und eine hier nicht näher interessierende Hülse in den freigeräumten Intradiskalraum eingesetzt. Durch die Hülse hindurch wird in die einander zugewandten Flächen der Wirbelkörper ein Gewinde eingeschnitten. Nun wird der Cage 10 mit dem Kopfabschnitt 12 voraus in die vorbereitete Öffnung eingeführt und mit dem Gewinde 42 zwischen die Wirbelkörper eingeschraubt. Die Länge des optimalen Cageelements 10 wurde zuvor beim Einschneiden des Gewindes in die Kontaktflächen der Wirbelkörper mit Hilfe einer Skala auf dem Gewindeschneider bestimmt.

Durch den konischen Abschnitt 22 des Einführrings 18 wird beim Einführen des Cageelements 10 die Verletzung von dem Einführweg benachbarten Gewebebereichen vermieden. Das Cageelement 10 wird so weit in die vorbereitete Öffnung eingeschraubt, bis es in der Endstellung mit der Stirnfläche des Verbindungselements 36 und deren Erweiterungen 38 und 40 sowie der Strebe 30 und dem entsprechenden Bereich des zylindrischen Abschnitts 20 am einen Wirbelkörper und mit den Kanten der Verbindungselemente 32 und 34 und der entsprechenden Streben 26 und 28 sowie dem entsprechenden Bereich des zylindrischen Abschnitts 20 am anderen Wirbelkörper anliegt (in Figur 4 sind die obere Fläche des unteren Wirbelkörpers durch eine untere gestrichelte Linie und die untere Fläche des oberen Wirbelkörpers durch eine obere gestrichelte Linie angedeutet).

Aufgrund der geringeren lichten Weite b im Bereich des Bodenabschnitts 16 im Vergleich zur lichten Weite a im Bereich des Einführrings 18 wird zwischen den beiden Wirbelkörpern wie gewünscht eine Lordose eingestellt. Aufgrund der "unrunden" Form insbesondere im Bereich des Bodenabschnitts 16 des Cageelements 10 ist darüber hinaus ein unbeabsichtigtes Sichverdrehen des Cageelements 10 aus der vom Operateur gewünschten Position so gut wie unmöglich.

Durch die hohe Flächenpressung an den Kanten der Streben 26 und 28 und der Verbindungselemente 34 und 34 graben sich diese im Laufe der Zeit in den Knochen ein, was das Cageelement 10 weiter in der gewünschten Position sichert. Nach dem Einsetzen des Cageelements 10 in den Intradiskalraum wird Spongiosa in den Intradiskalraum eingebracht. Da durch die Streben 26, 28 und 30 nur ein geringer Bereich der Umfangserstreckung des Cageelements 10 abgedeckt ist, ist von einem Wirbelkörper zum anderen durch das Spongiosagewebe hindurch ein sehr guter Kraftschluß möglich, wodurch die Knochenbildung zwischen den beiden Wirbelkörpern beschleunigt wird.

Das Cageelement 10 ist vorzugsweise aus nicht rostendem Stahl oder einem Kohlenstoff-Keramik-Material hergestellt.

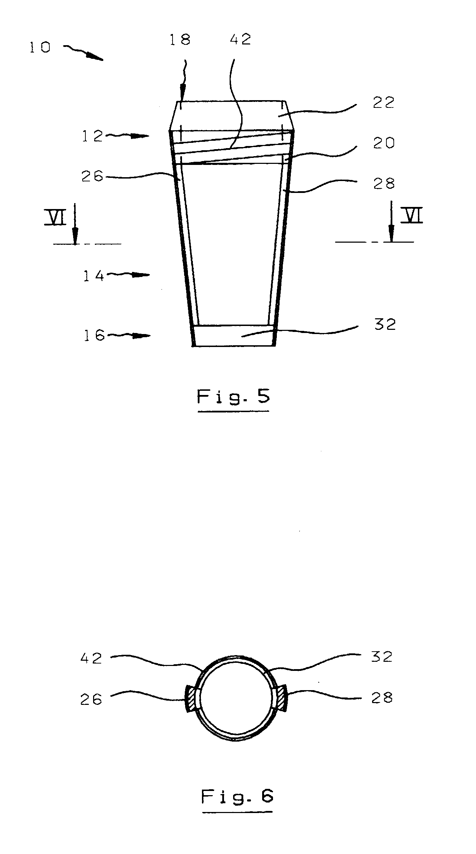

Nun wird auf das in den Figuren 5 und 6 dargestellte zweite Ausführungsbeispiel Bezug genommen. Elemente, welche die gleiche Funktion haben wie entsprechende Elemente bei dem in den Figuren 1 bis 4 dargestellten ersten Ausführungsbeispiel, tragen die gleichen Bezugszeichen.

Der Kopfabschnitt 12 des Cageelements 10 in Figur 5 weist ebenfalls einen oberen konischen Abschnitt 22 auf. Im Gegensatz zum ersten Ausführungsbeispiel ist aber auch der sich hieran nach unten anschließende Abschnitt 20 konisch ausgeführt, nämlich zu dem in Figur 5 unteren Ende hin sich verjüngend. An den konischen Abschnitt 20 sind zwei sich diametral gegenüberliegende Streben 26 und 28 angeformt, welche die sich verjüngende Kontur des Abschnitts 20 des Einführrings 18 zum Bodenabschnitt 10 hin fortsetzen. Der Bodenabschnitt 16 ist als Ring 32 ausgebildet und verbindet die Enden der Streben 26 und 28 miteinander. Auch er verjüngt sich nach unten hin, die durch den Abschnitt 20 des Einführrings 18 und die Streben 26 und 28 vorgegebene Kontur des Cageelements 10 fortführend. Auf der Außenfläche des Abschnitts 20, der Streben 26 und 28 und des Rings 32 ist schließlich ein Gewinde 42 vorhanden.

Das in den Figuren 5 und 6 dargestellte zweite Ausführungsbeispiel eines Cageelements 10 wird gleich eingesetzt wie das in den Figuren 1 bis 4 dargestellte erste Ausführungsbeispiel. Die Verwendung von nur zwei sich diametral gegenüberliegenden Streben 26, 28 hat den Vorteil, daß die durch die Streben 26, 28 abgedeckte Umfangserstreckung des Zentralabschnitts 14 äußerst klein ist. Dies kann noch dadurch optimiert werden, daß in einem nicht dargestellten Ausführungsbeispiel die Breite der Streben zum Bodenabschnitt hin kleiner wird. Der ringförmig ausgebildete Bodenabschnitt 16 erleichtert das Positionieren des Cageelements 10, allerdings ist das in den Figuren 5 und 6 dargestellte Cageelement 10 in seiner Einbaulage nicht so verdrehsicher wie das in Figuren 1 bis 4 dargestellte erste Ausführungsbeispiel.

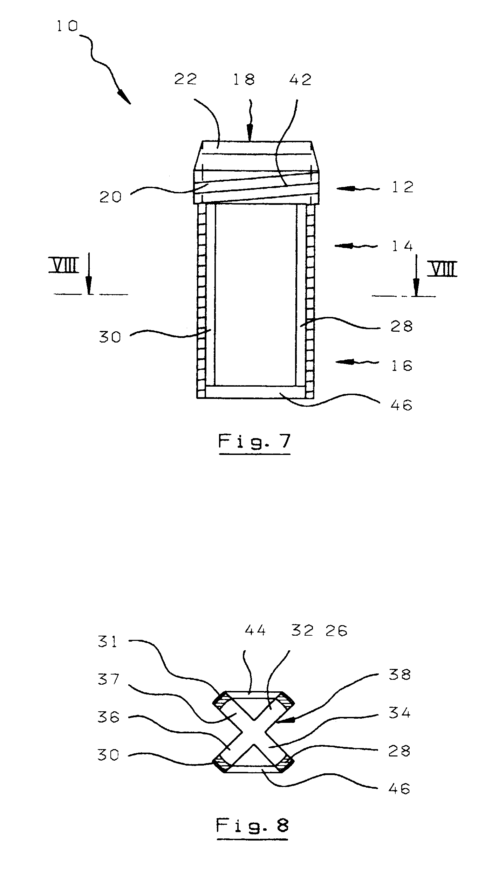

In den Figuren 7 und 8 ist noch ein drittes Ausführungsbeispiel eines Cageelements 10 dargestellt. Wie schon beim zweiten Ausführungsbeispiel in den Figuren 5 und 6 sind funktional entsprechende Elemente mit den gleichen Bezugszeichen wie bei dem in den Figuren 1 bis 4 dargestellten ersten Ausführungsbeispiel versehen.

Der Aufbau des in den Figuren 7 und 8 dargestellten Cageelements 10 entspricht im Bereich des Kopfabschnitts 12 exakt dem in den Figuren 1 bis 4 dargestellten ersten Ausführungsbeispiel. Der Zentralabschnitt 14 umfaßt jedoch vier Streben 26, 28, 30 und 31. Entsprechend umfaßt der Bodenabschnitt 16 vier Verbindungselemente 32, 34, 36 und 37, die ein Verbindungskreuz 38 bilden.

Im Gegensatz zu den beiden vorhergehenden Ausführungsbeispielen sind jedoch die Enden der Streben 26 und 31 durch ein Verbindungselement 44 miteinander verbunden, welches sich vom Ende der Strebe 26 in gerader Linie zum Ende der Strebe 31 erstreckt. Analog hierzu erstreckt sich vom Ende der Strebe 28 ein Verbindungselement 46 in gerader Linie bis zum Ende der Strebe 30 (vergleiche Figur 8).

Je nachdem, in welcher Winkellage das in den Figuren 7 und 8 dargestellte Cageelement 10 in den Intradiskalraum eingesetzt wird, dienen die Verbindungselemente 44 bzw. 46 dazu, eine große Auflagefläche für die entsprechenden Wirbelkörper bereitzustellen, oder um die Kraft von der dorsalen Zirkumferenz des einen Wirbelkörpers möglichst stabil und sicher zur dorsalen Zirkumferenz des anderen Wirbelkörpers leiten zu können.

## Patentansprüche

1. Cageelement mit einem ringförmigen Kopfabschnitt, einem an diesen angeformten Zentralabschnitt und einem wiederum an diesen angeformten Bodenabschnitt, dadurch gekennzeichnet, daß der Zentralabschnitt (14) von einer Mehrzahl sich axial vom Kopfabschnitt (12) bis zum Bodenabschnitt (16) erstreckender Streben (26, 28, 30) gebildet ist, die so schmal sind, daß die durch sie abgedeckte Umfangserstreckung maximal 25% der gesamten Umfangserstreckung des Zentralabschnitts (14) entspricht.

2. Cageelement nach Anspruch 1, dadurch gekennzeichnet, daß der Bodenabschnitt (16) sich im wesentlichen radial erstreckende Verbindungselemente (32, 34, 36) umfaßt, welche an ihrem radial äußeren Ende mit den Enden der Streben (26, 28, 30) und mit ihrem radial inneren Ende miteinander verbunden sind.

3. Cageelement nach Anspruch 2, dadurch gekennzeichnet, daß die Längsachsen der Verbindungselemente (32, 34, 36) die Längsachse des Cageelements (10) schneiden.

4. Cageelement nach einem der Ansprüche 2 oder 3, dadurch gekennzeichnet, daß es eine ungleiche Anzahl von Verbindungselementen aufweist, deren radial äußere Stirnflächen in Anlage an gegenüberliegende Wirbelkörper kommen können, wobei die Gesamtfläche der an dem einen Wirbelkörper anlegbaren radial äußeren Stirnflächen in etwa gleich der Gesamtfläche der an dem anderen Wirbelkörper anlegbaren radial äußeren Stirnflächen ist.

5. Cageelement nach Anspruch 4, dadurch gekennzeichnet, daß der Zentralabschnitt drei Streben (26, 28, 30) aufweist, und die radial äußere Stirnfläche eines Verbindungselements (36) in etwa gleich groß ist wie die Summe der radial äußeren Stirnflächen der beiden anderen Verbindungselemente (32, 34).

6. Cageelement nach einem der vorhergehenden Ansprüche, dadurch gekennzeichnet, daß der Bodenabschnitt (16) die Enden benachbarter Streben (26, 28, 30, 31) miteinander im wesentlichen geradlinig verbindende Verbindungselemente (44, 46) aufweist.

7. Cageelement nach einem der Ansprüche 1 bis 5, dadurch gekennzeichnet, daß der Bodenabschnitt (26) einen Bodenring (32) umfaßt, der mit den Enden der Streben (26, 28) verbunden ist.

8. Cageelement nach einem der vorhergehenden Ansprüche, dadurch gekennzeichnet, daß die kleinste lichte Außenweite (b) des Bodenabschnitts (16) des Cageelements (10) kleiner ist als diejenige (a) des Kopfabschnitts (12).

9. Cageelement nach einem der vorhergehenden Ansprüche, dadurch gekennzeichnet, daß auf einem zylindrischen Bereich (20) des Kopfabschnitts (12) ein Außengewinde (42) vorhanden ist.

10. Cageelement nach einem der vorhergehenden Ansprüche, dadurch gekennzeichnet, daß der Kopfabschnitt (12) an seinem den Streben (26, 28, 30) entgegengesetzten Ende einen nach außen konisch zulaufenden Bereich (22) aufweist.

11. Cageelement nach einem der vorhergehenden Ansprüche, dadurch gekennzeichnet, daß auf der radial außen liegenden Oberfläche der Streben (26, 28, 30) ein Außengewinde (42) vorhanden ist, so daß das Cageelement (10) insgesamt in eine Öffnung einschraubbar ist.

12. Satz von Cageelementen nach einem der vorhergehenden Ansprüche, dadurch gekennzeichnet, daß er Cageelemente unterschiedlichen Durchmessers und unterschiedlicher Länge umfaßt.
